(19) Europäisches Patentamt
European Patent Office
Office européen des brevets

(11) Veröffentlichungsnummer: **0 259 778**
**B1**

(12) # EUROPÄISCHE PATENTSCHRIFT

(45) Veröffentlichungstag der Patentschrift: **28.03.90**

(21) Anmeldenummer: **87112849.2**

(22) Anmeldetag: **03.09.87**

(51) Int. Cl.⁴: **C07C 233/53,** C12P 7/40, C12P 13/00, C12P 1/06, C12N 1/20, A61K 31/185 // (C12P7/40, C12R1:465, C12P1:06)

(54) **Antibiotisch wirksames Gentisinsäurederivat.**

(30) Priorität: **08.09.86 DE 3630521**

(43) Veröffentlichungstag der Anmeldung: **16.03.88 Patentblatt 88/11**

(45) Bekanntmachung des Hinweises auf die Patenterteilung: **28.03.90 Patentblatt 90/13**

(84) Benannte Vertragsstaaten: **AT BE CH DE FR GB IT LI NL SE**

(56) Entgegenhaltungen:
**DD-A- 94 394**

**Chemical Abstracts, Band 78, Nr. 23, 11. Juni 1973, Columbus, Ohio, USA M. FUJIMOTO "N-Gentisoylglutamic acid" Seite 349, Spalte 1, Zusammenfassung-Nr. 147 604u & JP-A-73-15853**

(73) Patentinhaber: **HOECHST AKTIENGESELLSCHAFT, Postfach 80 03 20, D-6230 Frankfurt am Main 80(DE)**

(72) Erfinder: **Zeeck, Axel Prof.-Dr., Brüder-Grimm-Allee 22, D-3400 Göttingen(DE)**
Erfinder: **Breiding-Mack, Sabine Dr., Papenberg 13, D-3381 Gross Döhren(DE)**
Erfinder: **Grabley, Susanne Dr., Hölderlinstrasse 7, D-6240 Königstein/Taunus(DE)**
Erfinder: **Voelskow, Hartmut Dr., Akazienstrasse 22, D-6234 Hatterheim am Main(DE)**
Erfinder: **Seibert, Gerhard Prof.-Dr., Gläserweg 21, D-6100 Darmstadt(DE)**

EP 0 259 778 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Aus einer Bodenprobe wurde ein Mikroorganismenstamm isoliert, der als Streptomyces species identifiziert und bei der Deutschen Sammlung von Mikroorganismen (DSM), 3400 Göttingen, Grisebachstr. 8, unter der Nummer DSM 3814 hinterlegt wurde. Der Stamm ist wie folgt charakterisiert:

| | |
|---|---|
| Sporenoberfläche: | Sp |
| Sporenmorphologie: | RF/RAa |
| Chromogenität: | M– |
| Luftmycelfarbe: | gelbgrau |

In einer eine Kohlenstoff- und Stickstoffquelle sowie die üblichen anorganischen Salze enthaltenden Nährlösung produzierte der Stamm DSM 3814 die antibiotisch wirksame 2-Acetylamino-gentisinsäure. Diese ist also erhältlich durch Fermentation des Stammes DSM 3814 und Isolierung aus dem Fermentationsmedium.

Die 2-Acetylamino-gentisinsäure wirkt antibakteriell, sowohl gegen gram-positive als auch gegen gram-negative Bakterien und kann daher in entsprechenden Heilmitteln zur Behandlung von bakteriell verursachten Infektionen an Mensch und Tier Verwendung finden.

Die Erfindung betrifft somit:

1) Die 2-Acetylamino-gentisinsäure der Formel I

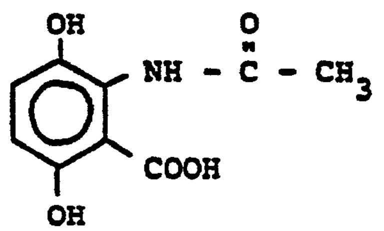

I

und deren Derivate, in denen unabhängig voneinander die Acetylgruppe an der 2-Aminogruppe durch $C_1$, $C_3$-$C_{10}$-Acyl ausgetauscht werden kann und die Carboxylgruppe in 3-Stellung am aromatischen Ring eine $C_1$-$C_{10}$-Alkylgruppe tragen kann.

2) Ein Verfahren zur Herstellung der Verbindung der Formel I, das dadurch gekennzeichnet ist, daß Streptomyces species DSM 3814 fermentiert wird bis sich die Verbindung der Formel I im Medium anhäuft.

3) Die Verwendung der Verbindung der Formel I und deren Derivate als aktive Komponente in Heilmitteln.

Im folgenden wird die Erfindung im einzelnen erläutert, bzw. in den Patentansprüchen definiert.

Anstelle des Stammes DSM 3814 können natürlich auch seine Mutanten und Varianten eingesetzt werden, soweit sie 2-Acetyl-amino-gentisinsäure produzieren. Solche Mutanten können in an sich bekannter Weise durch physikalische Mittel, beispielsweise Bestrahlung, wie mit Ultraviolett- oder Röntgenstrahlen, oder chemische Mutagene, wie beispielsweise Methansulfonsäureethylester (Ethylmethansulfonat, EMS) oder 2-Hydroxy-4-methoxy-benzophenon (MOB), erzeugt werden.

Als bevorzugte Kohlenstoffquellen für die aerobe Fermentation eignen sich assimilierbare Kohlenhydrate und Zuckeralkohole, wie Glukose, Laktose oder D-Mannit sowie kohlenhydrathaltige Naturprodukte, wie Malzextrakt. Als stickstoffhaltige Nährstoffe kommen in Betracht: Aminosäuren, Peptide und Proteine sowie deren Abbauprodukte, wie Peptone oder Tryptone, ferner Fleischextrakte, gemahlene Samen, beispielsweise von Mais, Weizen, Bohnen, Soja oder der Baumwollpflanze, Destillationsrückstände der Alkoholherstellung, Fleischmehle oder Hefeextrakte, aber auch Ammoniumsalze und Nitrate. An anderen anorganischen Salzen kann die Nährlösung beispielsweise Chloride, Carbonate, Sulfate oder Phosphate der Alkali- oder Erdalkalimetalle, Eisen, Zink und Mangan enthalten.

Die Bildung der 2-Acetylamino-gentisinsäure verläuft besonders gut in einer Nährlösung, die etwa 2 % Sojamehl und 2 % Mannit, jeweils bezogen auf das Gewicht der gesamten Nährlösung, enthält. Die Fermentation erfolgt aerob, also beispielsweise submers unter Schütteln oder Rühren in Schüttelkolben oder Fermentern, gegebenenfalls unter Einführen von Luft oder Sauerstoff. Die Fermentation kann in einem Temperaturbereich von etwa 18 bis 35°C, vorzugsweise bei etwa 25 bis 30°C, insbesondere bei 28 bis 30°C erfolgen. Der pH-Bereich sollte zwischen 6 und 8 liegen, vorteilhaft zwischen 6,5 und 7,5. Unter diesen Bedingungen zeigt die Kulturbrühe im allgemeinen nach 1 bis 4 Tagen eine nennenswerte antibiotische Wirkung.

Vorteilhaft kultiviert man in mehreren Stufen, d.h. man stellt zunächst eine oder mehrere Vorkulturen in einem flüssigen Nährmedium her, die dann in das eigentliche Produktionsmedium, die Hauptkultur, beispielsweise im Volumenverhältnis 1:10, überimpft werden. Die Vorkultur erhält man z.B., indem man ein versportes Mycel in eine Nährlösung überimpft und etwa 48 bis 72 Stunden wachsen läßt. Das versporte Mycel kann erhalten werden, indem man den Stamm etwa 7 Tage auf einem festen oder flüssigen Nährboden, beispielsweise Hefe-Malz-Agar, wachsen läßt.

Der Fermentationsverlauf kann anhand des pH-Wertes der Kultur, dem Mycelvolumen, durch Dünnschichtchromatographie oder Ausprüfen der biologischen Aktivität überwacht werden.

Die Isolierung der 2-Acetylamino-gentisinsäure aus dem Kulturmedium erfolgt nach bekannten Methoden unter Berücksichtigung der chemischen, physikalischen und biologischen Eigenschaften der Produkte. Zum Testen der Antibiotika-Konzentration im Kulturmedium oder in den einzelnen Isolierungsstufen kann die Dünnschichtchromatographie, beispielsweise auf Kieselgel mit n-Butanol/Essigsäure/Wasser als Laufmittel verwendet werden, wobei die Menge des gebildeten Antibiotikums zweckmäßig mit einer Eichlösung verglichen wird.

Das Antibiotikum kann aus der filtrierten Kulturbrühe durch Adsorption an bekannte Adsorberharze, beispielsweise Polystyrolharze ((R)Amberlite XAD 2), vorzugsweise bei pH 3 bis 7 abgetrennt und durch Elution mit einem polaren Lösemittel, z.B. Methanol oder Aceton, erhalten werden.

Die Reinisolierung erfolgt vorzugsweise an geeigneten Medien, wie Hydroxyalkoxypropyldextranen ((R)Sephadex LH-Marken), durch Elution mit einem niederen Alkanol, beispielsweise Methanol, oder einem Gemisch aus einem niederen Alkanol und einem weniger polaren organischen Lösemittel, z.B. Methanol/Ethylacetat und Sammeln der antibiotisch aktiven Fraktionen.

Die Substanz ist im festen Zustand und in Lösung im pH-Bereich von 3 bis 9, insbesondere von 5 bis 8, stabil. Die Verbindung läßt sich damit in übliche galenische Zubereitungen einarbeiten.

Eine chemische Derivatisierung der Verbindung kann nach an sich bekannte Methoden beispielsweise derart erfolgen, daß unabhängig voneinander die Acetylgruppe an der 2-Aminogruppe durch $C_1$ oder $C_3$-$C_{10}$-Acyl ausgetauscht werden kann und die Carboxylgruppe eine $C_1$-$C_{10}$-Alkylgruppe tragen kann, wobei die jeweiligen Kohlenwasserstoffreste gesättigt oder ungesättigt, geradkettig oder verzweigt oder zyklisch aliphatisch, aliphatisch-aromatisch oder aromatisch und unsubstituiert oder durch Halogene, z.B. Chlor oder Brom, bzw. durch veresterte oder veretherte Hydroxygruppen substituiert sein können.

Derartige Derivate zeigen ebenfalls antimikrobielle Aktivität.

Die antibakterielle Wirkung kommt sowohl gegen gram-positive als auch insbesondere gegen gram-negative Bakterien zur Geltung, wie beispielsweise im Platten-Agardiffusionstest in vitro (10 µl/Testrondell, 6 mm Durchmesser) gezeigt werden kann. Bei Probemengen von 1 µg pro Plättchen lassen sich Hemmhofdurchmesser von 16 mm, z.B. bei E. coli, finden.

In den folgenden Beispielen wird die Erfindung näher erläutert. Prozentangaben beziehen sich auf das Gewicht, wenn nichts anderes angegeben ist. Mischungsverhältnisse bei Flüssigkeiten beziehen sich auf das Volumen, sofern keine anderen Angaben gemacht sind.

Beispiel 1

a) Herstellung einer Sporensupension des Produzentenstammes DSM 3814

Mehrere Schrägröhrchen mit Malz-Hefeextrakt-Agar (4 g Hefeextrakt, 10 g Malzextrakt, 4 g Glukose, 20 g Agar zur Verfestigung, gelöst in 1 l dest. Wasser, pH 8,0 vor der Sterilisation eingestellt) werden mit Streptomyces spec., DSM 3814, beimpft und ca. 6 - 7 Tage bei 30°C inkubiert.

Die Sporen eines jeden Röhrchens werden mit je 3 ml Flüssigkeit [0,9 % NaCl und 0,1 % Polyoxyethylensorbitanmonooleat ((R)Tween 80) in dest. Wasser] abgeschwemmt. Die Suspensionen werden vereinigt und bis zur Beimpfung der Hauptkulturen bei 4°C aufbewahrt.

b) Herstellung einer Vorkultur des Produzentenstammes, DSM 3814, im Erlenmeyerkolben

5 Erlenmeyerkolben (300 ml Fassungsvermögen) mit je 100 ml Nährlösung (40 g Glukose, 30 g Sojamehl, 2,5 g NaCl, 2,5 g $CaCO_3$, gelöst in 1 l dest. Wasser, pH 7,5 vor der Sterilisation eingestellt) werden mit je 1,5 ml der möglichst frischen Sporensuspension (zum Beispiel 1a) angeimpft und auf einer Schüttelmaschine (180 UPM) bei 30°C 72 Stunden inkubiert.

c) Herstellung einer Hauptkultur des Produzentenstammes DSM 3814

Ein 300 ml Erlenmeyerkolben mit 100 ml Nährlösung (20 g Sojamehl, 20 g Mannit, gelöst in 1 l dest. Wasser, pH 7,5 vor der Sterilisation eingestellt) wird mit 3 ml der Vorkultur beimpft und bei 30°C auf der Schüttelmaschine (180 UPM) inkubiert.

Das Produktionsmaximum wird nach ca. 30 Stunden erreicht.

Die Ausbeuten von 2-Acetylamino-gentisinsäure liegen bei ca. 10 mg/l.

Beispiel 2

Fermentation des Produzentenstammes im Fermenter

Ein Fermenter mit einem Fassungsvermögen von 10 l wird unter folgenden Bedingungen betrieben: Bei 30°C Inkubationstemperatur und 600 UPM des Rührers werden ca. 7 l Luft pro Minute in die Kulturflüssigkeit (Medium wie in Beispiel 1c) eingeleitet. Mit 500 ml der Vorkultur (siehe Beispiel 1b) wird der Fermenter beimpft.
Das Produktionsoptimum wird nach ca. 30 Stunden erreicht. Die Kultur zeigt eine rosa-braune Färbung.
Die Ausbeuten von 2-Acetylamino-gentisinsäure liegen bei ca. 10 bis 50 mg/l.

Beispiel 3

Isolierung von 2-Acetylamino-gentisinsäure

Das Mycel, das man gemäß Beispiel 2 erhält, wird abgepreßt und verworfen, das Kulturfiltrat gefriergetrocknet und anschließend zweimal mit jeweils 2 l Methanol extrahiert. Die Methanol-Extrakte werden vereinigt und im Vakuum bis fast zur Trockne eingedampft. Den Rückstand suspendiert man in ca. 100 ml Methanol, gibt dann die gleiche Menge Ethylacetat hinzu, läßt Ungelöstes absitzen und chromatographiert den Überstand an einer Kieselgelsäule (30 x 10 cm) mit Ethylacetat/Methanol (4:1). Die das Antibiotikum enthaltenden Fraktionen werden vereinigt und 85 mg 2-Acetylamino-gentisinsäure nach einer weiteren Trennung an Sephadex LH 20 mit Methanol/Wasser (9:1;v:v) in reiner Form gewonnen. Das Produkt ist in Methanol löslich, jedoch nicht in Chloroform.
Es kann anhand der folgenden Daten identifiziert werden:

Dünnschichtchromatographie:

| Laufmittel: | | $R_F$-Werte |
|---|---|---|
| Essigsäureethylester/Methanol/Wasser (6:2:1) | | 0,7 |
| Essigsäureethylester/Methanol/Wasser (9:1:0,5) | | 0,2 |
| Essigsäureethylester/Methanol (4:1) | | 0,35 |
| UV: | $\lambda_{max}$ /nm/ = 340, 219<br>252 sh | in MeOH und MeOH + 2n HCl |
| | = 340, 223<br>252 sh | in MeOH + 2n NaOH |
| IR (KBr-Preßling): | /cm$^{-1}$/ 3470, 3050–2800, 1687, 1635 sh, 1600, 1562, 1478, 1356, 1306, 1271, 1233. | |
| $^1$H–NMR (200 MHz in $d_6$-DMSO): | δ = 2,19 (s, 3H); 6,49 (d, 9Hz, 1H); 6,76 (d, 9Hz, 1H); 9,76 (s, OH); 14,58 (s, breit, NH); 15,12 (s, OH) ppm | |
| $^{13}$C–NMR (50 MHz in $CD_3OD$): | δ = 24,0 ($CH_3$); 109,5 (q, C); 115,2 (CH); 124,3 (CH); 128,9 (q, C); 141,4 (q, C); 157,2 (q, C); 172,7 (CO); 176,3 (COOH) ppm | |

**Patentansprüche für die Vertragsstaaten: BE CH DE FR GB IT LI NL SE**

1. 2-Acetylamino-gentisinsäure der Formel I

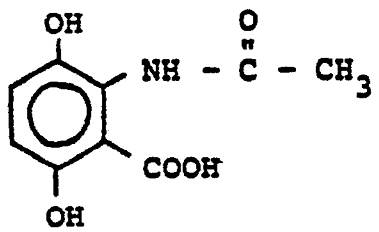

I

und deren Derivate in denen unabhängig voneinander die Acetylgruppe an der 2-Aminogruppe durch $C_1$, $C_3$-$C_{10}$-Acyl ausgetauscht ist und die Carboxylgruppe in der 3-Stellung am aromatischen Ring eine $C_1$-$C_{10}$-Alkylgruppe trägt.

2. Verfahren zur Herstellung der 2-Acetylamino-gentisinsäure der Formel I

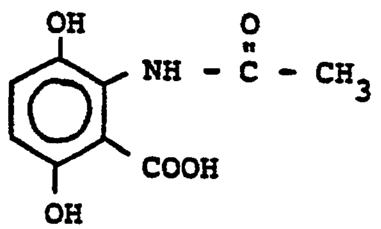

I

dadurch gekennzeichnet, daß Streptomyces spec., DSM 3814, kultiviert wird, bis sich de genannte Verbindung im Medium anhäuft.

3. Verfahren nach Anspruch 2, dadurch gekennzeichnet, daß Streptomyces spec., DSM 3814, in einer Nährlösung mit Sojamehl und Mannit kultiviert wird.

4. Verfahren nach Anspruch 2 oder 3, dadurch gekennzeichnet, daß bei einer Temperatur von 18 bis 35°C kultiviert wird.

5. Verfahren nach Anspruch 4, dadurch gekennzeichnet, daß bei einer Temperatur von 25 bis 30°C kultiviert wird.

6. Verfahren nach einem oder mehreren der Ansprüche 2 bis 5, dadurch gekennzeichnet, daß bei einem pH-Wert zwischen 6 und 8 kultiviert wird.

7. Verfahren nach Anspruch 6, dadurch gekennzeichnet, daß bei einem pH-Wert zwischen 6,5 und 7,5 kultiviert wird.

8. Verwendung der Verbindung der Formel I und deren Derivate nach Anspruch 1 zur Herstellung von Heilmitteln.

9. DSM 3814, seine Varianten und Mutanten, sofern sie die Verbindung der Formel I nach Anspruch 1 synthetisieren.

**Patentansprüche für den Vertragsstaat : AT**

1. Verfahren zur Herstellung der 2-Acetylamino-gentisinsäure der Formel I

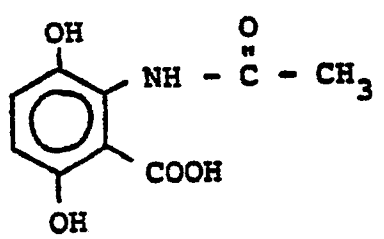

I

und deren Derivate, in denen unabhängig voneinander die Acetylgruppe an der 2-Aminogruppe durch $C_1$, $C_3$-$C_{10}$-Acyl ausgetauscht ist und die Carboxylgruppe in der 3-Stellung am aromatischen Ring eine $C_1$-$C_{10}$-Alkylgruppe trägt, dadurch gekennzeichnet, daß Streptomyces spec., DSM 3814, kultiviert wird, bis sich die Verbindung I im Medium anhäuft und diese gegebenenfalls derivatisiert wird.

2. Verfahren nach Anspruch 1, dadurch gekennzeichnet, daß Streptomyces spec., DSM 3814, in einer Nährlösung mit Sojamehl und Mannit kultiviert wird.

3. Verfahren nach Anspruch 1 oder 2, dadurch gekennzeichnet, daß bei einer Temperatur von 18 bis 30°C kultiviert wird.

4. Verfahren nach Anspruch 3, dadurch gekennzeichnet, daß bei einer Temperatur von 25 bis 30°C kultiviert wird.

5. Verfahren nach einem oder mehreren der Ansprüche 1 bis 4, dadurch gekennzeichnet, daß bei einem pH-Wert zwischen 6 und 8 kultiviert wird.

6. Verfahren nach Anspruch 5, dadurch gekennzeichnet, daß bei einem pH-Wert zwischen 6,5 und 7,5 kultiviert wird.

7. Verwendung der Verbindung der Formel I erhältlich nach Anspruch 1 und deren Derivate, in denen unabhängig voneinander die Acetylgruppe an der 2-Aminogruppe durch $C_1$, $C_3$-$C_{10}$-Acyl ausgetauscht werden kann und die Carboxylgruppe in 3-Stellung am aromatischen Ring eine $C_1$-$C_{10}$-Alkylgruppe tragen kann, zur Herstellung von Heilmitteln.

8. DSM 3814, seine Varianten und Mutanten, sofern sie die Verbindung der Formel I nach Anspruch 1 synthetisieren.

**Claims for the Contracting States: BE CH DE FR GB IT LI NL SE**

1. 2-Acetylaminogentisic acid of the formula I

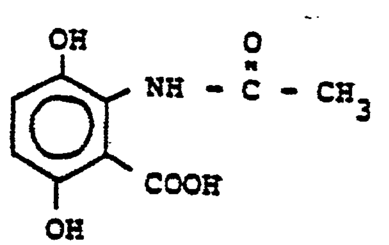

I

and its derivatives in which, independently of one another, the acetyl group on the 2-amino group is replaced by $C_1$ or $C_3$–$C_{10}$-acyl, and the carboxyl group in the 3-position on the aromatic ring carries a $C_1$–$C_{10}$-alkyl group.

2. A process for the preparation of 2-acetylaminogentisic acid of the formula I

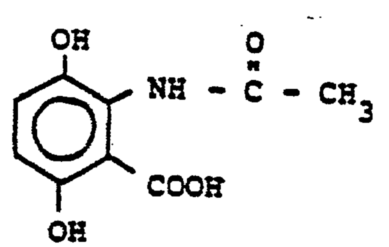

I

which comprises cultivating Streptomyces spec. DSM 3814 until the said compound accumulates in the medium.

3. The process as claimed in claim 2, wherein Streptomyces spec, DSM 3814 is cultivated in a nutrient solution containing soybean meal and mannitol.

4. The process as claimed in claim 2 or 3, wherein the cultivation is carried out at a temperature of 18 to 35°C.

5. The process as claimed in claim 4, wherein the cultivation is carried out at a temperature of 25 to 30°C.

6. The process as claimed in one or more of claims 2 to 5, wherein the cultivation is carried out at a pH between 6 and 8.

7. The process as claimed in claim 6, wherein the cultivation is carried out at a pH between 6.5 and 7.5.

8. The use of the compound of the formula I and its derivatives as claimed in claim 1 for the preparation of medicines.

9. DSM 3814 and its variants and mutants which are able to synthesize the compound of the formula I as claimed in claim 1.

**Revendications pour les Etats contractants: BE CH DE FR GB IT LI NL SE**

1. Acide 2-acétylamino-gentisique de formule I

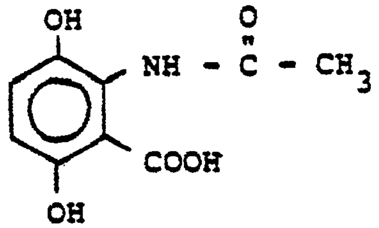

I

et ses dérivés dans lesquels, indépendamment l'un de l'autre, le groupe acétyle sur le radical 2-amino est remplacé par un groupe acyle en $C_1$, $C_3$–$C_{10}$, et le groupe carboxy en position 3 sur le noyau aromatique porte un groupe alkyle en $C_1$–$C_{10}$.

2. Procédé pour la préparation de l'acide 2-acétylamino-gentisique de formule I

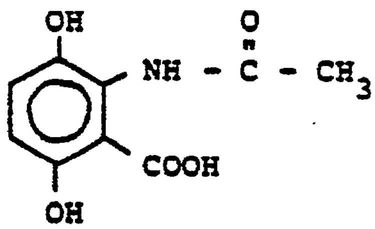

I

caractérisé en ce que l'on cultive Streptomyces sp. DSM 3814 jusqu'à ce que le composé cité s'accumule dans le milieu.

3. Procédé selon la revendication 2, caractérisé en ce que l'on cultive Streptomyces sp. DSM 3814 dans une solution nutritive contenant de la farine de soja et du mannitol.

4. Procédé selon la revendication 2 ou 3, caractérisé en ce que l'on effectue la culture à une température de 18 à 35°C.

5. Procédé selon la revendication 4, caractérisé en ce que l'on effectue la culture à une température de 25 à 30°C.

6. Procédé selon une ou plusieurs des revendications 2 à 5, caractérisé en ce que l'on effectue la culture à un pH entre 6 et 8.

7. Procédé selon la revendication 6, caractérisé en ce que l'on effectue la culture à un pH entre 6,5 et 7,5.

8. Utilisation du composé de formule 1 et de ses dérivés selon la revendication 1, pour la fabrication de médicaments.

9. DSM 3814, ses variants et mutants, dans la mesure où ils synthétisent le composé de formule I selon la revendication 1.

**Claims for the Contracting State: AT**

1. A process for the preparation of 2-acetylaminogentisic acid of the formula I

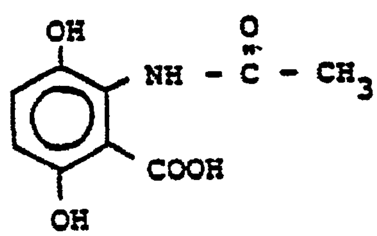

I

and its derivatives in which, independently of one another, the acetyl group on the 2-amino group is replaced by $C_1$ or $C_3$–$C_{10}$-acyl, and the carboxyl group in the 3-position on the aromatic ring carries a $C_1$–$C_{10}$-alkyl group, which comprises cultivating Streptomyces spec. DSM 3814 until the compound I accumulates in the medium, and derivatizing said compounds is where appropriate.

2. The process as claimed in claim 1, wherein Streptomyces spec. DSM 3814 is cultivated in a nutrient

solution containing soybean meal and mannitol.

3. The process as claimed in claim 1 or 2, wherein the cultivation is carried out at a temperature of 18 to 30°C.

4. The process as claimed in claim 3, wherein the cultivation is carried out at a temperature of 25 to 30°C.

5. The process as claimed in one or more of claims 1 to 4, wherein the cultivation is carried out at a pH between 6 and 8.

6. The process as claimed in claim 5, wherein the cultivation is carried out at pH between 6.5 and 7.5.

7. The use of the compound of the formula I obtainable as claimed in claim 1 and its derivatives in which, independently of one another, the acetyl group on the 2-amino group can be replaced by $C_1$ or $C_3$–$C_{10}$-acyl, and the carboxyl group in the 3-position on the aromatic ring can carry a $C_1$–$C_{10}$-alkyl group, for the preparation of medicines.

8. DSM 3814 and its variants and mutants which are able to synthesize the compound of the formula I as claimed in claim 1.

**Revendications pour l'Etat contractant: AT**

1. Procédé pour la préparation de l'acide 2-acétylamino-gentisique de formule I

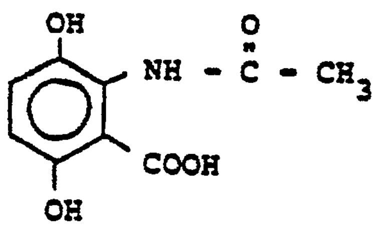

I

et de ses dérivés dans lesquels, indépendamment l'un de l'autre, le groupe acétyle sur le radical 2-amino est remplacé par un groupe acyle en $C_1$, $C_3$–$C_{10}$, et le groupe carboxy en position 3 sur le noyau aromatique porte un groupe alkyle en $C_1$–$C_{10}$, caractérisé en ce que l'on cultive Streptomyces sp. DSM 3814 jusqu'à ce que le composé I s'accumule dans le milieu, et on transforme éventuellement celui-ci en dérivé.

2. Procédé selon la revendication 1, caractérisé en ce que l'on cultive Streptomyces sp. DSM 3814 dans une solution nutritive contenant de la farine de soja et du mannitol.

3. Procédé selon la revendication 1 ou 2, caractérisé en ce que l'on effectue la culture à une température de 18 à 35°C.

4. Procédé selon la revendication 3, caractérisé en ce que l'on effectue la culture à une température de 25 à 30°C.

5. Procédé selon une ou plusieurs des revendications 1 à 4, caractérisé en ce que l'on effectue la culture à un pH entre 6 et 8.

6. Procédé selon la revendication 5, caractérisé en ce que l'on effectue la culture à un pH entre 6,5 et 7,5.

7. Utilisation du composé de formule I pouvant être obtenu selon la revendication 1, et de ses dérivés dans lesquels, indépendamment l'un de l'autre, le groupe acétyle sur le radical 2-amino peut être remplacé par un groupe acyle en $C_1$, $C_3$–$C_{10}$ et le groupe carboxy en position 3 sur le noyau aromatique peut porter un groupe alkyle en $C_1$–$C_{10}$, pour la fabrication de médicaments.

8. DSM 3814, ses variants et mutants, dans la mesure où ils synthétisent le composé de formule I selon la revendication 1.